(19) Europäisches Patentamt
European Patent Office
Office européen des brevets

(11) Publication number: 0 149 594
A2

(12) # EUROPEAN PATENT APPLICATION

(21) Application number: 85870006.5

(22) Date of filing: 15.01.85

(51) Int. Cl.⁴: C 12 P 21/02
C 07 K 5/06

(30) Priority: 16.01.84 US 571037

(43) Date of publication of application:
24.07.85 Bulletin 85/30

(84) Designated Contracting States:
AT BE CH DE FR GB IT LI LU NL SE

(71) Applicant: Monsanto Company
Patent Department 800 North Lindbergh Boulevard
St. Louis Missouri 63167(US)

(72) Inventor: Likos, John Joseph
9837 Hudson Avenue
St. Louis Missouri 63119(US)

(72) Inventor: Glover, George Irvin
22 Wind Rush Creek West
Creve Coeur Missouri 63141(US)

(74) Representative: Lunt, John Cooper et al,
Monsanto Europe S.A. Patent Department Avenue de
Tervuren 270-272 Letter Box No 1
B-1150 Brussels(BE)

(54) **Enzymatic coupling of n-formyl amino acids and/or peptide residues.**

(57) An improved process for enzyme mediated peptide bond formation is disclosed. The process embraces the discovery that the formyl group can be utilized to selectively protect the amino group of one substrate from undergoing peptide bond formation.

EP 0 149 594 A2

Croydon Printing Company Ltd.

# ENZYMATIC COUPLING OF N-FORMYL AMINO ACIDS AND/OR PEPTIDE RESIDUES

## BACKGROUND OF THE INVENTION

This invention relates to an enzymatic method for the synthesis of dipeptide or polypeptide alkyl esters and more particularly to a method in which N-formyl protected aspartic acid and a lower alkyl ester of phenylalanine are reacted in aqueous solvent using a protease to produce a dipeptide such as α-L-aspartyl-L-phenylalanine methyl ester.

Prior work in dipeptide synthesis has been generally directed towards the preparation of α-L-aspartyl-L-phenylalanine alkyl esters, particularly the methyl ester, known for their usefulness as sweetening agents. For example, U. S. Patent 3,786,039 discloses a method in which lower alkyl esters of L-phenylalanine are reacted directly in inert organic solvents with L-aspartic anhydride having its amino group protected by a formyl, carbobenzoxy, or the p-methoxycarbobenzoxy group which is removed after the reaction to yield the desired α-L-aspartyl-L-phenylalanine ester. U.S. Patent 3,933,721 discloses a process comprising reacting an N-protected-L-aspartic anhydride with L-phenylalanine to form N-protected-α-L-aspartyl-L-phenylalanine in which the N-protecting group is selected from the group consisting of formyl, acetyl, benzoyl, substituted and unsubstituted carbobenzoxy, t-butoxycarbonyl and hydrohalide

salts. The amino protecting group is subsequently removed to form α-L-aspartyl-L-phenylalanine alkyl ester. U. S. Patent 3,879,372 discloses a method with high coupling efficiencies and increased α-isomer selectivity wherein aspartyl amino acid alkyl esters are prepared by reacting N-protected aspartic acid anhydride and an α-amino acid lower alkyl ester in an essentially aqueous solvent. The N-protecting group selected from the group consisting of formyl, carbobenzoxy, t-butyloxycarbonyl, t-amyloxycarbonyl, p-methoxybenzyloxycarbonyl, and the trifluoroacetyl group is then removed from the reaction product.

A disadvantage generally shared by chemical synthesis methods is racemization of the carboxyl component of the C-terminal amino acid residue. In addition, peptide bond synthesis by chemical synthesis utilizing N-blocked aspartic acid yields both α and β isomers. When racemization and unwanted isomer formation occurs, the purity of the product is decreased and it is necessary to separate the unwanted forms from the desired product. As a result, chemical synthesis of dipeptides is not considered commercially attractive.

To circumvent the aforementioned disadvantage, methods have been developed to enzymatically direct the peptide bond formation. For example, U. S. Patent 4,086,136 discloses a method for producing an N-protected polypeptide ester which comprises the use of a thiol proteinase or serine proteinase enzyme in aqueous solution. The amino protecting group is selected from the group consisting of tertiary alkoxy and benzyloxy, benzylamino and benzylhydrylamino which can be

substituted with an inert substituent. U. S. Patent 4,284,721 discloses a method for producing a dipeptide from an N-protected aspartic acid and a phenylalanine lower alkyl ester comprising the use of a water-containing immobilized metallo-proteinase such as thermolysin. The amino protecting group is selected from the group consisting of benzyloxycarbonyl, p-methoxybenzyloxycarbonyl, and t-butoxycarbonyl. In general, therefore, previous methods of enzymatically coupling amino acids have required a relatively complex and expensive amino protecting group.

It is, therefore, the overall object of the present invention to provide an improved enzymatic method for the preparation of dipeptides.

Accordingly, it is an object of the present invention to provide an enzymatic method comprising the use of a relatively inexpensive amino protecting group.

It is still another object of the present invention to provide an enzymatic method wherein the amino protecting group is easily removed.

It is yet another object of the present invention to provide an enzymatic method wherein the amino protecting group is recoverable.

These and other objects, features, and advantages of the present invention will become apparent to those skilled in the art from the following description.

SUMMARY OF THE INVENTION

The present invention provides an enzymatic process for preparing dipeptide or polypeptide alkyl esters which comprises reacting an N-formyl protected amino acid with an amino acid alkyl ester in the presence of a suitable protease to form the N-formyl protected dipeptide alkyl ester. The formyl protecting group is subsequently removed to form the dipeptide alkyl ester. Although the process of this invention is capable of coupling essentially any two amino acids and/or peptide segments, the preferred embodiment is described with respect to the preparation of α-L-aspartyl-L-phenylalanine lower alkyl ester, particularly α-L-aspartyl-L-phenylalanine methyl ester known for its use as a sweetening agent.

DESCRIPTION OF THE PREFERRED EMBODIMENTS

The present invention embraces the surprising discovery that the formyl group can be effectively utilized as an amino protecting group for protease mediated dipeptide and polypeptide syntheses. It has been previously reported that N-substituted dipeptides having small protecting groups, such as the formyl group, bind poorly to protease enzymes. As described above, prior methods of carrying out protease mediated peptide synthesis were limited to relatively complex and expensive amino protecting groups. While no detectable peptide coupling occurred using the N-formyl protected amino acids at concentrations taught for other protected amino acids, it was unexpectedly found that the formyl group could be effectively used as an amino protecting

group if the concentration of the N-formyl amino acid was sufficiently elevated. The present invention in its broadest aspect embraces the use of the amino protecting formyl group in an enzymatic process for peptide bond formation between amino acids and/or peptides.

Amino acid coupling, according to the present invention, proceeds according to the following preferred reactions:

(1)

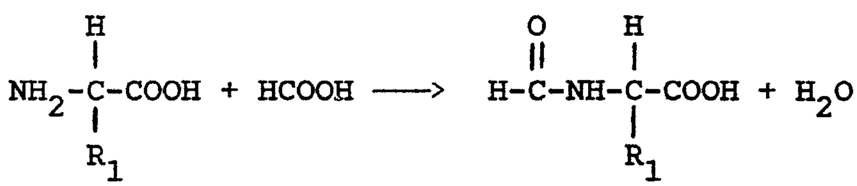

(2)

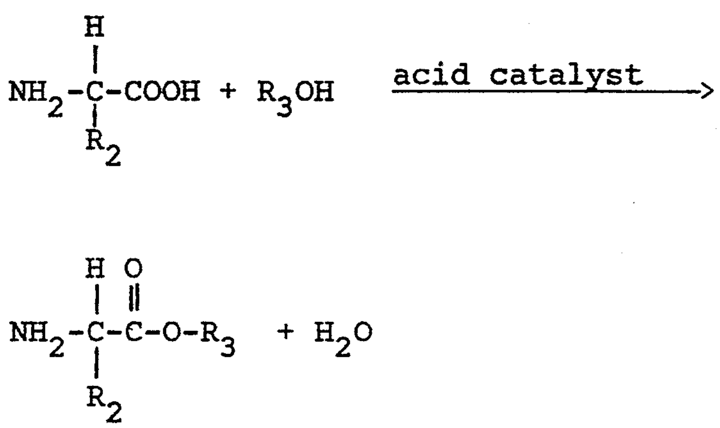


(3)

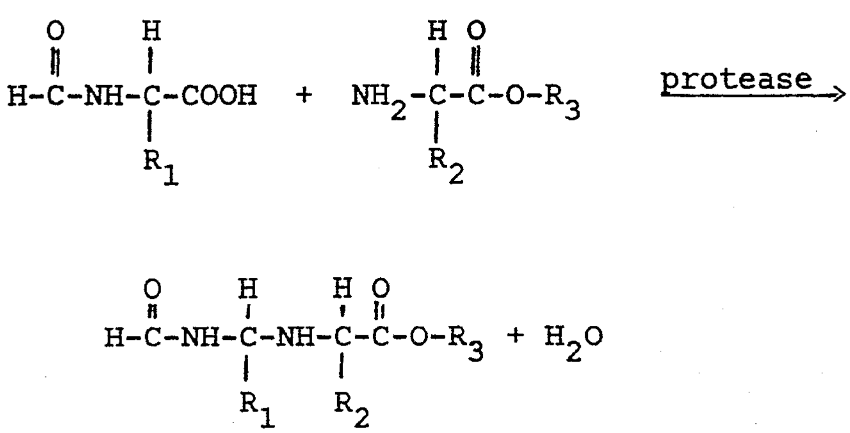

(4) 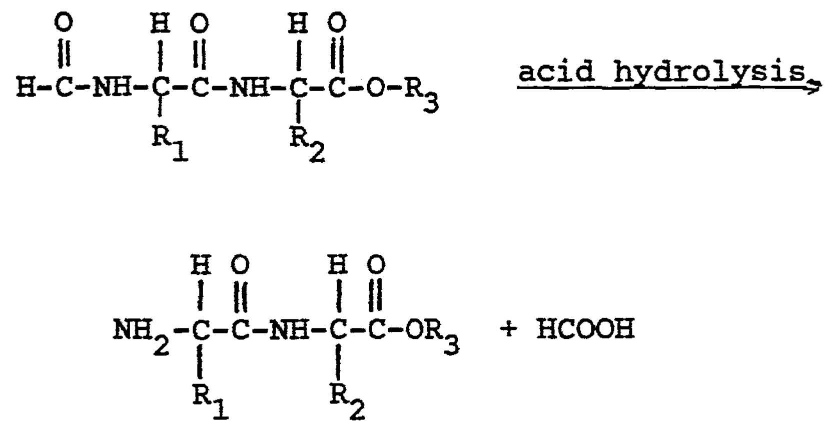


in which $R_1$ and $R_2$ are selected so that the amino acids are naturally occurring amino acids such as phenylalanine, aspartic acid, leucine, alanine and the like. $R_3$ is selected from the group consisting of branched and unbranched alkyl radicals such as methyl, ethyl, n-propyl, isopropyl, n-butyl, isobutyl and the like.

As shown in equation (1) a first amino acid is reacted with formic acid to form the N-formyl amino acid derivative. While the amino protecting formyl group can be added by heating the amino acid in concentrated formic acid, use of an acylating agent such as acetic anhydride is preferred. Using an acylating agent affords the advantage of being able to carry out the reaction at a lower temperature and thereby minimize the danger of racemization of optically active L-amino acid preparations. A second amino acid, which may be the same or different from the first amino acid, is reacted with an alkanol in the presence of an acid catalyst as shown in equation (2) to form the amino acid alkyl ester derivative. Suitable acid catalysts include, but are not limited to, hydrogen halides and sulfuric acid.

While use of the ester derivative is preferred to aid binding of the amino acid to the protease, it is not believed to be critical and it is expected that, in some instances, the free amino acid will perform as well as the alkyl ester derivatives. Moreover, since peptide segments bind relatively well to protease enzymes, use of the alkyl ester is not required.

The two above-described amino acid derivatives are reacted in the presence of a protease enzyme known to hydrolyze the peptide linkage between the two amino acids. Since the action of the protease enzyme is reversible, there will be a mixture of the N-formyl dipeptide and uncoupled amino acid derivatives upon equilibrium of the reaction represented by equation (3) above. The dipeptide is separated from the unreacted amino acid derivatives by conventional separation means such as selective extraction from the mixture. The amino protecting formyl group is easily removed by mild hydrolysis with a dilute acid. Formic acid recovered from the above-described protecting group removal process can be easily recycled to the first step in the process of the present invention.

Examples of suitable protease enzymes are listed in the following table along with their corresponding sites of action. The coupling reaction of equation (3) is maintained at a pH within the operable limits of the protease selected and preferably near the optimum pH to optimize enzyme activity.

TABLE

| Protease | Major Sites of Action | Optimal pH |
|---|---|---|
| Chymotrypsin | Trp, Phe, Tyr | 7-8 |
| Bromelain | Arg, Lys, Gly | 5-8 |
| Ficin | Arg, Lys, Gly | 6.5 |
| Papain | Arg, Lys, Gly | 6-7 |
| Pepsin | Trp, Phe, Tyr Met, Leu | 2-3 |
| Subtilisin | Aromatic & Aliphatic Residues | 7-8 |
| Thermolysin | Amino-linked bonds of Aliphatic residues | 6.5-8.5 |
| Trypsin | Arg, Lys | 7-8 |

While the alkyl esters embraced by the present invention are considered noncritical, lower alkyl esters are preferred and the methyl ester is particularly preferred since methanol is readily available and relatively inexpensive. By "lower alkyl esters" is meant those obtained by using an alkanol have between one and four carbon atoms, inclusive. If the free acid form is desired the alkyl substituent can be removed by saponification. The free acid is easily obtained from the salt formed by saponification by reacidification of the mixture.

While the present invention is described above with respect to coupling single amino acids derivatives to produce the corresponding dipeptide, it is evident to those skilled in the art that

polypeptides can be prepared by the process of the present invention. To prepare a polypeptide one would need only to take two peptide segments making up the desired polypeptide, prepare the appropriate formyl derivative according to the reaction described by equation (1) and then react the two peptide segments in the presence of a suitable protease. By "peptide" is meant a compound consisting of at least two amino acid residues bonded together through a peptide linkage. The coupled polypeptide can be removed from unreacted substrate peptides by methods such as gel filtration and ion exchange. The formyl protecting group can be removed as described above. The peptide segments can be stepwise synthesized by sequential applications of the process of the present invention or other means described by Greenstein et al. in Chemistry of the Amino Acids , Vol. 2, Wiley, New York (1961). It should be understood, however, that the protease used to couple the peptide segments may also hydrolyze certain peptide linkages within the individual peptide segments. The protease should be selected to minimize hydrolysis of the segments. It should be evident to those skilled in the art that this may not be possible in all situations.

The following examples are included to better illustrate the practice of the present invention. It should be understood that these examples are included for illustrative purposes only and is not, in any way, intended to limit the scope of the present invention.

## EXAMPLE 1

N-formylaspartic acid and phenylalanine methyl ester hydrochloride were purchased from Sigma Chemical Company, St. Louis, Missouri. An aqueous 5 ml reaction mixture consisting of 0.85 g (5.3 mmole) N-formyl-L-aspartic acid, 1.15 g (5.3 mmole) L-phenylalanine methyl ester hydrochloride and 40 mg thermolysin was adjusted to pH 6.5 with aqueous sodium hydroxide. The amino acid substrates were present at a concentration of approximately 1.0 M. After twenty-four hours at 25$^{o}$C. the resulting slurry was centrifuged yielding 1.5 g (wet weight) of white solid which was identified as the (1:1) addition product of N-formyl-L-aspartyl-L-phenylalanine methyl ester and L-phenylalanine methyl ester. The solid was suspended in water, the pH adjusted to 1.6 with aqueous hydrochloric acid and the dipeptide product was extracted with ethyl acetate. Evaporation of the ethyl acetate yielded about 200 mg of N-formyl-α-L-aspartyl-L-phenylalanine methyl ester. The dipeptide product was identified by comparison of thin-layer chromatographic behavior, HPLC retention time and NMR spectrum of chemically synthesized N-formyl-α-L-aspartyl-L-phenylalanine methyl ester.

EXAMPLE 2

The same experiment was conducted as described in Example 1 except that the aqueous 5 ml reaction mixture consisted of 1.61 g (10 mmole) N-formyl-L-aspartic acid, 0.43 g (2 mmole) L-phenylalanine methyl ester hydrochloride and 100 mg of thermolysin and was adjusted to pH 5.8 with aqueous sodium hydroxide. After two and one-half hours at 35°C the resulting slurry was centrifuged, the solids washed one time with water and then freeze dried. This yielded 0.47 g (0.9 mmole) of the (1:1) addition product of N-formyl-L-aspartyl-L-phenylalanine methyl ester and L-phenylalanine methyl ester. This represents a yield of 90% which is comparable with 84% conversion as measured by HPLC analysis of the reaction mixture.

EXAMPLE 3

The same experiment was conducted as described in Example 2 except that an aqueous 10 ml reaction mixture consisted of 1.61 g (10 mmole) N-formyl-L-aspartic acid, 3.59 g (20 mmole) L-phenylalanine methyl ester and 200 mg of thermolysin and was adjusted to pH 6.6 with aqueous sodium hydroxide. After three and one-half hours at 35°C the resulting slurry was centrifuged, the solids washed one time with water and then freeze dried. This yielded 1.9 g (3.8 mmole) of the (1:1) addition product of N-formyl-L-aspartyl-L-phenylalanine methyl ester and L-phenylalanine methyl ester. This represents a yield of 38% which is comparable with 41% conversion as measured by HPLC analysis of the reaction mixture.

CLAIMS:

1. In a process in which anenzyme is used to mediate peptide bond formation between two substrates selected from the group consisting of amino acids and peptides, the improvement comprising selectively protecting the amino group of one substrate from undergoing said bond formation by using either the N-formyl amino acid derivative or N-formyl peptide derivative.

2. The process of Claim 1 in which the other substrate is an alkyl ester derivative.

3. The process of Claim 1 in which the enzyme is a protease.

4. The process of Claim 2 in which the ester is a lower alkyl ester.

5. A process for producing N-formyl-α-L-aspartyl-L-phenylalanine alkyl ester which comprises reacting N-formyl-L-aspartic acid with L-phenylalanine alkyl ester in the presence of an enzyme known to mediate peptide bond formation between L-aspartic acid and L-phenylalanine and then acidifying the mixture to yield the dipeptide product.

6. The process of Claim 5 in which the L-phenylalanine ester is a lower alkyl ester.

7. The process of Claim 5 in which the L-phenylalanine ester is the methyl ester.

8. The process of Claim 5 in which the enzyme is thermolysin.

9. A process for producing α-L-aspartyl-L-phenylalanine methyl ester which comprises reacting N-formyl-L-aspartic acid with L-phenylalanine methyl ester in the presence of an enzyme known to mediate peptide bond formation between L-aspartic acid and L-phenylalanine methyl ester and then subjecting the N-formyl dipeptide to mild hydrolysis to remove the amino protecting formyl group.

10. The process of Claim 9 in which the enzyme is thermolysin.